# EUROPEAN PATENT SPECIFICATION

(11) **EP 1 768 549 B1**
(45) Date of publication and mention of the grant of the patent: **20.05.2009**
(21) Application number: 05745516.4
(22) Date of filing: 01.06.2005
(51) Int. Cl.: A61B 5/022, A61M 5/155

(54) **A DEVICE FOR PRESSURIZATION**
VORRICHTUNG ZUR DRUCKBEAUFSCHLAGUNG
DISPOSITIF DE PRESSURISATION

(30) Priority: 24.06.2004 SE 0401634
(43) Date of publication of application: 04.04.2007
(73) Proprietor: QF Medtech AB, 171 77 Stockholm (SE)
(72) Inventor: HILLBORG, Olle, S-170 73 Solna (SE); PERSSON, Stefan, S-184 32 Åkersberga (SE)
(74) Representative: Miller, James Lionel Woolverton
(86) International application number: PCT/SE2005/000830
(87) International publication number: WO 2006/001744

(56) References cited:
- US-A- 5 792 061
- US-A- 5 824 000
- US-A1- 2003 208 158

## Description

The present invention relates to an object pressurizing device of the kind defined in the preamble of claim 1.

US 4088126 teaches a device for measuring blood pressure, wherein the device includes an inflatable sleeve for restricting the flow of blood in the arm of a patient for instance, wherein the sleeve is connected to a pressure gauge, and wherein a hand pump is connected to the sleeve via a line and including an air inlet having a check valve, wherein the device also includes a manually actuable air release valve for controlled release of air from the sleeve. According to US 4088126, a compressed air pump driven by an electric motor can be connected to the air inlet of the hand pump. The electric motor is connected to the mains network and includes a control means which can be manoeuvred with the foot of an operator.

US-A-5792061 discloses a pressurizing device having an electrical pump, pedal pump or compressed air cylinder as well as a hand-held pump for adjusting the inflation of a pressure cuff to a minute degree.

In a hospital or medical care environment it is normally required that the device can be moved readily, between different places and that it can be readily used at said places. The requirement of access to an electric socket connected to the mains network and the transportation difficulties associated with an electrically driven compressed air pump with associated foot switch limits the usefulness of this known device.

Accordingly, an object of the present invention is to provide a device in which the sleeve can be alternatively inflated from a compressed gas network that is normally found in a hospital or care environment and which normally has a compressed air outlet available at a large number of places in the wards and rooms of the hospital.

This object is achieved by the invention.

The invention is defined in the accompanying claim 1.

Further embodiments of the invention are made apparent in the accompanying dependent claims.

The invention enables the user to use the hand pump to inflate the sleeve, in a conventional manner. When a compressed gas outlet is available, the user can readily connect the plug to the pressurizing gas outlet and control inflation of the sleeve through the medium of a corresponding manually actuable valve on the box. Even though the box is connected to the pressure gas outlet, the user is able to use the hand pump to pressurize the sleeve, if he/she so desires.

In addition to being used to measure the blood pressure of a patient, the inventive device can also be used to pressurize a flexible container, for instance a bag containing an infusion (infusion solution and/or blood) which shall be infused into a patient from the bag at an overpressure that can be set with the device, for instance via a hose connected to the bag and provided with a cannula that is inserted into a blood vessel of the patient.

The pressure gas source may, alternatively, consist in a pressure gas bottle. Such pressure gas bottles are often carried on boats, for instance patient transporting vehicles, thereby enabling an inventive device to be used on boats or in vehicles.

Particularly preferred embodiments of the inventive device will now be described by way of example with reference to the accompanying drawing, in which
Fig. 1 is a diagrammatic illustration of a device according to the present invention applied to the upper arm of a patient with the intention of measuring his/her blood pressure;
Fig. 2 illustrates the device applied to an infusion bag whose content shall be infused into a patient; and
Fig. 3 illustrates a connection between a box and a hand pump included in the device.

An inflatable sleeve 2 is shown applied around the patient's arm 1 and connected to a pressure gauge 3. A hand-operated balloon-type air pump 30 includes an air inlet opening 31 which includes a check valve 32. The hand pump 30 is connected to the interior of the sleeve 2 for inflating said sleeve, via a line 52, a box 5 and a hose 4, so that the sleeve is able to restrict the flow of blood in the arm 1. An air release valve 53 is connected to the outlet line 52 of the hand pump 30 and includes a manually actuable element 55 which, when activated allows air to be released from the interior of the sleeve 2 through an outlet 54, via the line 4, the box 5 and the line 52.

The box 5 has an outlet connection 51 to which the hose 4 is connected. The box 5 also includes a manually controllable inlet valve 6 for the input of compressed air from a pressure gas source to the interior of the box 5. The pressure gas source consists in a pressure gas network that has been installed in a hospital environment and which has an accessible socket outlet 20 mounted on a wall 21, such outlet sockets being found in many rooms of the hospital. A hose 8 is provided with a connector 10 that can be plugged into the wall socket 20. The hose 8 connects to the valve 6. As shown, the hose 8 includes a restriction 9 for setting an upper limit at which the flow of gas can be passed to the interior of the box 5. The hose 8 is shown connected to the valve 6 via a check valve 7. It will be understood by the person of average skill in this art that the restriction 9 and the check valve 7 may be given other placements in the flow path between the wall outlet 20 and the interior of the box 5, although the preferred placement is on the box.

The box may include an emergency or over pressure valve 58 which functions to release air automatically from the box 5 should the pressure in the box exceed a pre-determined value. The flow through the restriction 9 will, of course, be lower than the flow that can depart through the emergency or safety valve 58.

The connecting piece 10 on the hose 8 is conveniently adapted for co-action with the wall mounted outlet 20 so as to hold a closure valve open when in engagement with said outlet 20.

The hose 8 may be releasably connected to the check valve 7. The person of average skill in this art will realize that the input valve 6 and the check valve 7 can swap places in the air inflow path to the box 5. The pressure gas source 20 is normally a terminal to a pressurized breathing gas network. Alternatively, the pressure gas source may comprise a pressure gas bottle. Such pressure gas bottles are often carried in vehicles and on boats, particularly in ambulances and other patient transporting vehicles, therewith enabling the inventive device to be used also in such vehicles and boats.

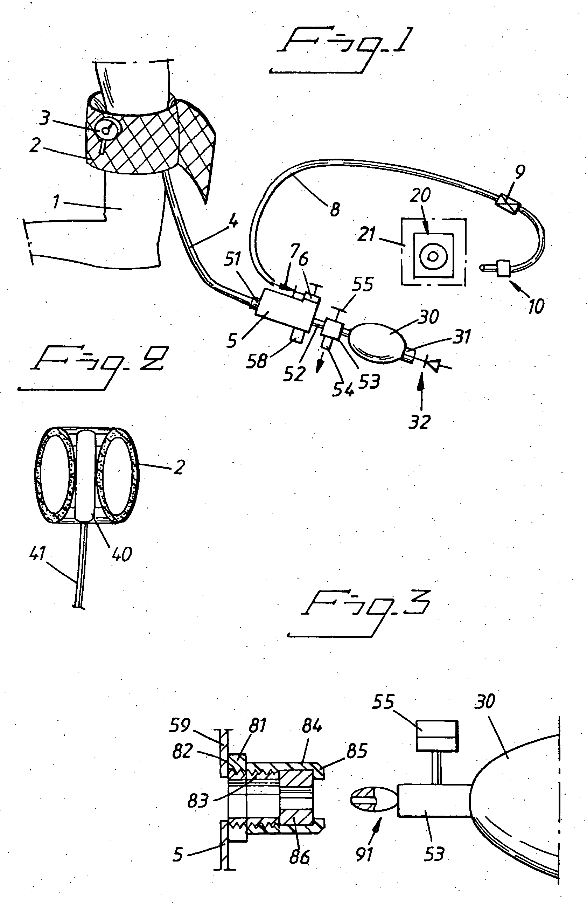

Figure 2 illustrates the inflatable sleeve 2 surrounding a bag 40 of infusion solution, which can thereby be infused into a patient at a settable overpressure, which may be desirable in certain instance in order to maintain or to strengthen the infusion supply, for instance, via the hose 41 connected to the bag 40 and for instance via a cannula (not shown) connected to the hose and receivable in a blood vessel of the patient.

Figure 3 illustrates the valve 53 which is connected to the pumping balloon 30 and which includes an elongate, externally oval and longitudinally projecting tubular part 91. The wall of the box 5 includes a channel in which a tubular rubber bush 86 is clamped. The through-flow channel of the bush 86 is smaller than the maximum diameter of the tubular part 91 and serves to accommodate said part, wherewith the change in the shape of the bush 86 can be taken up by the ability of the rubber bush to deform elastically. As will be seen from figure 3, the bush 86 is tensioned axially in an internal groove that extends circumferentially in a tubular stud which projects out from the wall of the box 5. It will be seen that a nut 81 is firmly connected to the outside of the wall 59 around a medium through-flow opening therein. An externally threaded bush 83 is firmly screwed into the nut 81 and supports at its free end against the rubber bush 86. A plastic sleeve 84 is placed externally on the bush 83 and projects beyond the bush in an axial direction and includes radially bent end edges 85. The rubber bush 86 is clamped axially between the radially bent end edges 85 of the sleeve 84 and the outer end of the bush 83. The part 91 is accommodated firmly in the rubber bush 86 although being releasable therefrom, and is able to swing to some extent in the bush while retaining a tight connection therewith. The part 91 can be readily pulled out of the bush 86 manually when necessary, and can be readily fitted to the bush 86.

In practice, the sleeve 84 together with the bush 86 can be placed on the inside of the box. In addition, the sleeve and its ring element that enclose the bush radially and axially may be produced in some other way, for instance by injection moulding or some technically equivalent method.

## Claims

1. A device for pressurizing an inflatable sleeve (2), wherein the device comprises a pressure gauge connected to the sleeve (2), a hand pump (30) which is connected to the sleeve (2) via a line (52;4) and which includes an air inlet (31) having a check valve (32), wherein the device further comprises a manually actuable air release valve (53) for the controlled release of gas from the sleeve interior, wherein a box (5) is connected in the line (52,4) between the sleeve (2) and the air release valve (53); and wherein the box (5) can be connected to a pressure gas source (20) by means of a hose (8), wherein the hose (8) connects with the interior of the box (5) via a check valve (7) and a manually controllable valve (6) for the input of gas from the pressure gas source to the sleeve (2) via the box (5).

2. A device according to claim 1 **characterised in that** the pressure gas source includes an outlet (20) for a gas distribution system in a hospital, wherein the gas is preferably pressurized breathing air; and **in that** the hose (8) that can be connected to the pressure gas source (20) includes a connecting piece (10) that co-acts with the outlet (20) such as to open a closure valve therein and therewith permit pressurized gas to flow through the hose (8).

3. A device according to claim 1 or claim 2, **characterized in that** a restriction (9) for limiting the flow of gas from the pressure gas source is connected in the flow path between the pressure gas source and the interior of the box (5).

4. A device according to claim 1 or 2 or 3, **characterized by** an overpressure valve (58) for limiting the interior gas pressure of the device to a pre-determined value in an upward sense.

5. A device according to any one of claims 1-4, **characterized in that** the valve (6) for the input of gas from the pressure gas source is biased towards a closed state.

6. A device according to any one of claims 1-5, **characterized in that** the box-associated check valve (7) is mounted in the box (5).

7. A device according to claims 1 or 6, in combination with a pressure gas bottle that preferably contains pressurized breathing air and that can be transported by a patient transporting vehicle, as the pressure gas source.

8. A device according to any one of claims 1-7, **characterized in that** the sleeve (2) is adapted for application to the arm of a patient in order to measure the patients blood pressure, or **in that** the sleeve is intended for application to a bag that contains infusion solution and/or blood, for pressurizing the same and diffusing the solution and/or blood into a patient.

9. A device according to any one of claims 1-8, **characterized in that** the box includes an inlet channel in which a tubular rubber brush (86) is clamped; and **in that** the hand pump includes an outlet line (53) having an outwardly projecting oval line-part (91) which can be forced into the through-flow channel of the rubber bush (86) for releasable and conductible attachment.

## Patentansprüche

1. Vorrichtung zum Druckbeaufschlagen einer aufblasbaren Manschette (2), wobei die Vorrichtung ein Druckmessgerät, das mit der Manschette (2) verbunden ist, und eine Handpumpe (30) umfasst, die mit der Manschette (2) über eine Leitung (52; 4) verbunden ist und einen Lufteinlass (31) mit einem Rückschlagventil (32) enthält, wobei die Vorrichtung weiters ein manuell betätigbares Luftablassventil (53) zum gesteuerten Ablassen von Gas aus dem Inneren der Manschette umfasst, wobei ein Gehäuse (5) an der Leitung (52, 4) zwischen der Manschette (2) und dem Luftablassventil (53) angeschlossen ist; und wobei das Gehäuse (5) über einen Schlauch (8) mit einer Druckgasquelle (20) verbunden werden kann, wobei der Schlauch (8) das Innere des Gehäuses (5) über ein Rückschlagventil (7) und ein manuell steuerbares Ventil (6) zum Einlass von Gas aus der Druckgasquelle in die Manschette (2) über das Gehäuse (5) verbindet.

2. Vorrichtung nach Anspruch 1, **dadurch gekennzeichnet, dass** die Druckgasquelle einen Auslass (20) für ein Gasverteilungssystem in einem Krankenhaus enthält, wobei das Gas vorzugsweise mit Druck beaufschlagte Atemluft ist; und dass der Schlauch (8), der mit der Druckgasquelle (20) verbunden werden kann, ein Verbindungsstück (10) enthält, das mit dem Auslass (20) zusammenwirkt, um ein Verschlussventil in diesem zu öffnen und somit dem mit Druck beaufschlagten Gas das Strömen durch den Schlauch (8) zu ermöglichen.

3. Vorrichtung nach Anspruch 1 oder 2, **dadurch gekennzeichnet, dass** eine Beschränkung (9) zum Begrenzen des Gasstroms aus der Druckgasquelle am Strömungsweg zwischen der Druckgasquelle und dem Inneren des Gehäuses (5) angeschlossen ist.

4. Vorrichtung nach Anspruch 1, 2 oder 3, **gekennzeichnet durch** ein Überdruckventil (58) zum Begrenzen des Innengasdrucks der Vorrichtung auf einen vordefinierten Wert in eine Aufwärtsrichtung.

5. Vorrichtung nach einem der Ansprüche 1 bis 4, **dadurch gekennzeichnet, dass** das Ventil (6) für den Gaseinlass aus der Druckgasquelle hin zu einem geschlossenen Zustand vorgespannt ist.

6. Vorrichtung nach einem der Ansprüche 1 bis 5, **dadurch gekennzeichnet, dass** das dem Gehäuse zugeordnete Rückschlagventil (7) im Gehäuse (5) befestigt ist.

7. Vorrichtung nach Anspruch 1 oder 6 in Kombination mit einer Druckgasflasche, die vorzugsweise mit Druck beaufschlagte Atemluft enthält und über ein Patienttransportvehikel als Druckgasquelle transportiert werden kann.

8. Vorrichtung nach einem der Ansprüche 1 bis 7, **dadurch gekennzeichnet, dass** die Manschette (2) eingerichtet ist, um am Arm eines Patienten zur Messung des Blutdrucks des Patienten angelegt zu werden, oder dass die Manschette an einen Beutel angelegt werden soll, der die Infusionslösung und/oder Blut enthält, zum Druckbeaufschlagen der Infusionslösung und/oder des Bluts und zum Diffundieren der Lösung und/oder des Bluts in einen Patienten.

9. Vorrichtung nach einem der Ansprüche 1 bis 8, **dadurch gekennzeichnet, dass** das Gehäuse einen Einlasskanal enthält, in den eine rohrförmige Gummimuffe (86) geklemmt ist; und dass die Handpumpe eine Auslassleitung (53) mit einem nach außen vorstehenden, ovalen Leitungsteil (91) enthält, der zur lösbaren und leitfähigen Befestigung in den Durchflusskanal der Gummimuffe (86) gezwungen werden kann.

## Revendications

1. Dispositif de pressurisation d'un manchon gonflable (2), le dispositif comprenant un manomètre relié au manchon (2), une pompe à main (30) qui est reliée au manchon (2) via une conduite (52 ; 4) et qui comprend une entrée d'air (31) ayant un clapet anti-retour (32), dans lequel le dispositif comprend en outre un détenteur d'air sous pression (53) pouvant être actionné manuellement pour assurer la libération contrôlée du gaz de l'intérieur du manchon, une boîte (5) étant reliée à la conduite (52, 4) entre le manchon (2) et le détenteur d'air sous pression (53) ; et dans lequel la boîte (5) peut être raccordée à une source de gaz pressurisé (20) au moyen d'un tuyau (8), où le tuyau (8) relie l'intérieur de la boîte (5) via un clapet anti-retour (7) et un clapet pouvant être contrôlé manuellement (6) pour l'entrée de gaz de la source de gaz pressurisé dans le manchon (2) via la boîte (5).

2. Dispositif selon la revendication 1, **caractérisé en ce que** la source de gaz pressurisé comprend une sortie (20) d'un système de distribution de gaz dans un hôpital, le gaz étant de préférence de l'air respirable pressurisé ; et **en ce que** le tuyau (8) qui peut être raccordé à la source de gaz pressurisé (20) comprend un élément de raccordement (10) qui coopère avec la sortie (20) de façon à ouvrir un clapet de fermeture dans celle-ci et à permettre avec celle-ci l'écoulement du gaz pressurisé dans le tuyau (8).

3. Dispositif selon la revendication 1 ou la revendication 2, **caractérisé en ce qu'**un élément de restriction (9) servant à limiter l'écoulement du gaz de la source de gaz pressurisé est relié dans la voie d'écoulement entre la source de gaz pressurisé et l'intérieur de la boîte (5).

4. Dispositif selon la revendication 1 ou 2 ou 3, **caractérisé par** un clapet de surpression (58) servant à limiter la pression de gaz intérieure du dispositif à une valeur prédéterminée dans un sens ascendant.

5. Dispositif selon l'une des revendications 1 à 4, **caractérisé en ce que** le clapet (6) pour l'entrée de gaz de la source de gaz pressurisé est incliné vers une position fermée.

6. Dispositif selon l'une quelconque des revendications 1 à 5, **caractérisé en ce que** le clapet anti-retour associé à la boîte (7) est monté dans la boîte (5).

7. Dispositif selon les revendications 1 ou 6, associé à une bouteille de gaz pressurisé qui contient de préférence de l'air respirable pressurisé et qui peut être transportée par un véhicule transportant un patient en tant que source de gaz pressurisé.

8. Dispositif selon l'une des revendications 1 à 7, **caractérisé en ce que** le manchon (2) est adapté pour être appliqué au bras d'un patient afin de mesurer la pression sanguine du patient ou **en ce que** le manchon est destiné à être appliqué à une poche qui contient une solution de perfusion et/ou du sang, pour mettre celle-ci sous pression et diffuser la solution et/ou le sang dans un patient.

9. Dispositif selon l'une quelconque des revendications 1 à 8, **caractérisé en ce que** la boîte comprend un canal d'entrée dans lequel une brosse en caoutchouc tubulaire (86) est fixée ; et **en ce que** la pompe à main comprend une conduite de sortie (53) ayant une partie de conduite ovale se projetant vers l'extérieur (91) qui peut être insérée dans le canal d'écoulement de la brosse en caoutchouc (86) pour constituer une fixation amovible et conductrice.
